# FASCICULE DE BREVET EUROPEEN

(11) **EP 1 656 131 B1**
(45) Date de publication et mention de la délivrance du brevet: **02.03.2011**
(21) Numéro de dépôt: 04737684.3
(22) Date de dépôt: 08.07.2004
(51) Int. Cl.: A61K 31/205, A61K 31/616, A61P 9/00, A61P 9/14, A61P 9/10, A61P 7/10, A61P 37/02, A61P 15/10

(54) **UTILISATION DE LA BETAINE POUR LE TRAITEMENT DE LA CLAUDICATION INTERMITTENTE**
VERWENDUNG VON BETAIN ZUR BEHANDLUNG VON CLAUDICATIO INTERMITTENS
USE OF BETAINE FOR TREATING INTERMITTENT CLAUDICATION

(30) Priorité: 15.07.2003 BE 200300408
(43) Date de publication de la demande: 17.05.2006
(73) Titulaire: Messadek, Jallal, 4000 Liège (BE)
(72) Inventeur: Messadek, Jallal, 4000 Liège (BE)
(86) Numéro de dépôt international: PCT/BE2004/000101
(87) Numéro de publication internationale: WO 2005/004854

(56) Documents cités:
- WO-A-00/25764
- WO-A-95/15750
- WO-A-98/19690
- WO-A-02/062322
- WO-A-2004/049095
- FR-A- 2 119 889
- US-A- 5 716 941
- US-A1- 2002 183 380
- "BETAINE FOR HOMOCYSTINURIA" MEDICAL LETTER ON DRUGS AND THERAPEUTICS, NEW ROCHELLE, NY, US, vol. 39, no. 993, 31 janvier 1997 (1997-01-31), page 12, XP000853853 ISSN: 0025-732X
- MALINOW M R (REPRINT): "PLASMA HOMOCYST(E)INE AND ARTERIAL OCCLUSIVE DISEASES - A MINIREVIEW" CLINICAL CHEMISTRY, (JAN 1995) VOL. 41, NO. 1, PP. 173-176. ISSN: 0009-9147., janvier 1995 (1995-01), XP008039186
- AL-AWAMI M ET AL: "Homocysteine levels in patients with Raynaud's phenomenon" VASA - JOURNAL OF VASCULAR DISEASES 2002 SWITZERLAND, vol. 31, no. 2, 2002, pages 87-90, XP008039171 ISSN: 0301-1526
- DIDIER A -F ET AL: "Distal cutaneous necrosis, an unusual etiology: Hyperhomocysteinemia" ANNALES DE DERMATOLOGIE ET DE VENEREOLOGIE, vol. 126, no. 11, novembre 1999 (1999-11), pages 822-825, XP008039169 ISSN: 0151-9638
- STAMMLER F ET AL: "Prevalence of hyperhomocysteinaemia in thrombangiitis obliterans (Buerger's disease): Does homocysteine play a pathogenetic role?" DMW (DEUTSCHE MEDIZINISCHE WOCHENSCHRIFT), vol. 121, no. 46, 1996, pages 1417-1423, XP008039176 ISSN: 0012-0472
- HIATT WILLIAM R: "Medical treatment of peripheral arterial disease and claudication" NEW ENGLAND JOURNAL OF MEDICINE, vol. 344, no. 21, 24 May 2001 (2001-05-24) , pages 1608-1621, ISSN: 0028-4793
- R. A. MANGIAFICO: "Pharmacotherary for intermittent claudication: from consensus-based to evidence-based treatment" VASCULAR DISEASE PREVENTION, vol. 1, 2004, pages 1-15,
- NORVIT TRIAL INVESTIGATORS: "Homocysteine lowering and cardiovascular events after acute myocardial infarction" NEW ENGLAND JOURNAL OF MEDICINE, vol. 354, no. 15, April 2006 (2006-04), pages 1578-1588, ISSN: 0028-4793
- LONN EVA ET AL: "Homocysteine lowering with folic acid and B vitamins in vascular disease" NEW ENGLAND JOURNAL OF MEDICINE, vol. 354, no. 15, April 2006 (2006-04), pages 1567-1577, ISSN: 0028-4793
- DUPREZ D ET AL: "Estimation of walking distance in intermittent claudication: Need for standardization" EUROPEAN HEART JOURNAL, vol. 20, no. 9, May 1999 (1999-05), pages 641-644, ISSN: 0195-668X
- Retrieved from the Internet: URL:http://www.umm.edu/patiented/articles/ intermittent_claudication_other_symptoms_o f_peri...>

## Description

La présente invention décrit l'utilisation de la glycine bétaine ou d'un sel thérapeutiquement actif de celle-ci en tant que principe thérapeutiquement actif pour la préparation d'une composition pharmaceutique pour le traitement de la claudication intermittente. La présente invention décrit également une composition à usage oral pour le traitement de la claudication intermittente, ladite composition comprenant comme principe actif unique une quantité thérapeutiquement active de glycine bétaine par dose unitaire. Plus particulièrement l'invention décrit un médicament destiné à traiter un malade souffrant d'une claudication intermittente causée par des désordres circulatoires périphériques tels que l'artériosclérose oblitérante ou la thrombo-angéite oblitérante.

### Principe de l'invention

L'artérite est une inflammation des artères. Anatomiquement, les lésions inflammatoires peuvent toucher une ou plusieurs des tuniques vasculaires soit endartérites, mésartérites, périartérites ou même tout le vaisseau et c'est la panatérite.

Les artérites peuvent être diffuses à tout l'organisme, localisées à un territoire vasculaire (artérite des membres inférieurs) ou même limitées à un seul vaisseau (l'artère temporale dans la maladie de Horton) ou à des portions d'un vaisseau (périartérite noueuse). Théoriquement il existe deux groupes d'artérites : les artérites aiguës et les artérites chroniques. En pratique trois grandes variétés d'artérites peuvent être définies:
1. Les artérites infectieuses, soit aiguës (rickettsioses) soit subaiguës qui sont des panartérites ;
2. Les artérites dégénératives, qui sont surtout des endartérites ou des mésartérites qui atteignent le sujet âgé, provoquées par une sclérose (artériosclérose, artériolosclérose) ou des dépôts lipidiques (athérome) ;
3. Les artérites crypto-génétiques (de cause inconnue, attribuées parfois à des troubles immunologiques), qui sont habituellement des collagénoses (périartérite noueuse, lupus érythématheux aigu disséminé, angéite de Wegener, maladie de Buerger, artérite temporale, etc.).

Les complications anatomiques provoquées par les lésions artérielles, quelle qu'en soit la cause sont au nombre de deux :
- l'affaiblissement de la paroi artérielle qui est à l'origine d'une distension, d'un anévrisme avec son risque de rupture ;
- l'épaississement de la paroi artérielle qui diminue la lumière du vaisseau favorisant la thrombose.

La distension artérielle (anévrisme) et la thrombose entraînent une mauvaise vascularisation dans les territoires irrigués par ces vaisseaux cause possible d'atrophie et d'infarctus. Comme on le voit la thrombose dans ce cas ci n'est qu'une des manifestations de l'artérite qui se situe en tant qu'événement en amont de celle-ci.

Dans ce cas précis également la thrombose est provoquée par des forces mécaniques (shear stress) entraînant l'agrégation plaquettaire suite à l'activation des plaquettes et l'homocystéine n'ayant aucun effet causatif.

L'artérite se manifeste au niveau des membres inférieurs par la claudication intermittente, une douleur de type crampe des masses musculaires du mollet, apparaissant à l'effort (habituellement la marche) et cédant au repos. La douleur apparaît après une distance parcourue appelée périmètre de marche dont l'importance diminue lorsque la maladie s'aggrave, rendant alors toute marche impossible. Lorsque les douleurs persistent même au repos, il faut redouter l'apparition d'une gangrène, complication majeure de l'artérite. La gangrène apparaît comme une plaque d'abord violacée puis noirâtre, qui peut s'étendre rapidement et nécessiter l'amputation. Le diagnostic d'artérite est évoqué devant le refroidissement d'un membre, la diminution ou même l'abolition des battements artériels, la diminution ou la disparition des oscillations que l'on recherche avec un oscillomètre de Pachon. L'artériographie est indispensable pour préciser l'état exact du tronc artériel. Devant toute artérite il faut chercher le diabète sucré car les artérites sont fréquentes chez les diabétiques et se compliquent volontiers de gangrène.

L'artériopathie oblitérante chronique est due à la formation de plaques d'athérome au sein de la paroi artérielle. Le dépôt athéromateux est une lésion irréversible qui atteint les trois couches de la paroi artérielle. Celle-ci voit son calibre diminuer progressivement au décours de la maladie, jusqu'à induire une ischémie du territoire irrigué par l'artère. En fait, l'artériopathie des membres inférieurs est une manifestation tardive de l'athérosclérose: il est courant que ces patients aient également des atteintes athérosclérosiques des artères coronaires, des carotides et de l'aorte abdominale. Si le processus à l'origine du développement de la plaque d'athérome reste méconnu, les facteurs de risque sont bien connus: le tabac, l'hypertension artérielle, le diabète, l'hypercholestérolémie (+/- l'augmentation du rapport LDL/HDL).

Les varices sont également des manifestations cliniques des artérites et sont des dilatations anormales et permanentes des veines superficielles. L'insuffisance de la valvule ostiale au niveau de la crosse de la saphène joue un rôle essentiel dans le développement des varices des veines sous-jacentes. L'augmentation de la pression dans les veines superficielles et la stase sanguine qui en découle peuvent être à l'origine de troubles trophiques.

La claudication veineuse est également une manifestation des artérites, de même que le syndrome de Raynaud.

La claudication intermittente signifie qu'un symptôme dans lequel deux conditions se suivent et se répètent: la difficulté de marche continue entraînant le malaise et les douleurs musculaires des membres inférieurs causés après la locomotion d'une distance constante et l'allégement de ces symptômes ou la possibilité de pouvoir de nouveau se mouvoir sans douleur après un repos de plusieurs minutes. La claudication intermittente artérielle correspond à une douleur ischémique d'effort des membres inférieurs secondaire à une artériopathie chronique oblitérante. La douleur est liée à une insuffisance relative des apports sanguins d'un groupe musculaire par rapport à un surcroît de demande métabolique qui conduit à l'acidose.

Les artérites induisent également la nécrose de différents tissus et organes. L'invention a pour but l'utilisation thérapeutique ou pharmaceutique de la glycine bétaine de formule (CH₃)₃ N⁺ (CH₂) COO⁻ et ses sels pharmaceutiquement acceptables afin de traiter la claudication intermittente.

Dans un aspect les bétaines lipidiques, les combinaisons et les formulations telles que décrites dans PCT/ IB 02/ 04923 de l'inventeur pourront être utilisées. Dans un autre aspect particulier l'utilisation d'une bétaine en combinaison avec des cellules souches est divulguée.

L'utilisation de la glycine bétaine en tant que principe actif ou pharmaceutique pour le traitement et/ou la prévention d'artérite(s) dégénératives est décrite. Par exemple, on a remarque que la glycine bétaine était appropriée pour réduire ou limiter la vitesse de développement de dégénérescence d'artérite(s) dégénérative(s), voire d'arrêter ou bloquer le développement de dégénérescence d'artérite(s) dégénérative(s), ou encore mieux voire la rémission au moins partielle de dégénérescence d'artérite(s) dégénérative(s).

### Etat de la technique

### Traitements

Les traitements utilisés actuellement pour les artérites peuvent être les vasodilatateurs qui sont souvent insuffisants. Les anticoagulants oraux tels que la warfarine retardent l'apparition des thromboses. Les antibiotiques jugulent l'infection de la gangrène. Le traitement chirurgical peut être nécessaire en cas de non efficacité de ces traitements.

Le traitement médicamenteux est indiqué en l'absence d'amélioration significative de la claudication après 6 mois de prise en charge ou chez les patients trop âgés pour bénéficier d'une rééducation standardisée. Ces traitements ne se sont pas avérés statistiquement efficaces pour réduire, voire limiter la vitesse de dégénérescence d'artérite(s) dégénérative(s), et donc encore moins pour arrêter ou bloquer la dégénérescence.

Dans le cas particulier de la claudication intermittente les médicaments utilisés sont par exemple, la pentoxifylline qui a l'effet d'améliorer la déformabilité des globules rouges et de là la viscosité du sang, le naftidrofuryl et le cilostazol qui possède une activité anti-agrégante et vasodilatatrice. Ces agents ont montré une certaine efficacité clinique mais leur usage reste lié à des effets secondaires non négligeables. Les héparines de bas poids moléculaire, la ticlopidine, le clopidogrel et l'aspirine ont été également testés avec des résultats variables.

On connaît diverses utilisations thérapeutiques de la bétaine notamment celles décrites dans les documents suivants :

US 6, 008, 221 et US 6, 127, 370 décrivent un traitement de la maladie d'Alzheimer où on combine un acide folique avec une bétaine afin de réduire des taux d'homocysteine. Le traitement décrit également l'utilisation de telles combinaisons afin de réduire des événements micro vasculaires pouvant induire des neuro- dégénérations. La claudication intermittente est décrite. Dans ces demandes on enseigne l'utilisation de la bétaine par voie orale, mais en quantité extrêmement faible, notamment dans la revendication 12 où la dose journalière de bétaine proposée varie entre 0,1 à 100 mg , quantité beaucoup trop faible pour pouvoir attendre-un quelconque effet thérapeutique.

WO 00 /25764 décrit une combinaison comprenant un donneur de méthyle, une vitamine et un bioflavonoide afin de réduire l'homocysteine et notamment les maladies associées à l'hyper homocysteinémie. Les auteurs en listant les maladies associées à l'hyper homocysteinémie font référence à la claudication intermittente sans pour autant qu'il n'y ait un exemple spécifique ni un lien scientifique clairement établi entre ces deux pathologies. De plus ce document décrit dans son unique exemple une composition orale destinée à une prise unique, ladite composition contenant 600 mg de bétaine, 0,5 mg de sel calcium d'acide L-5-Methyle tétra hydrofolique et 500 mg d'Isoquercetine. L'utilisation de bioflavonoides ainsi que de vitamines en plus de la bétaine n'apporte rien au point de vue thérapeutique dans le traitement de la claudication intermittente, de plus s'agissant d'une composition destinée à un usage unique la quantité de bétaine utilisée pour chaque prise unique ne permet pas d'atteindre des concentrations thérapeutiques nécessaires de bétaine au vu de la faible biodisponibilité absolue par voie orale de cette dernière. Les composés revendiqués dans ce document par l'apport d'ingrédients non effectifs dans la claudication intermittente, pourraient compromettre l'efficacité de la bétaine dans cette pathologie et n'apportant pas les quantités nécessaires de bétaine pour un effet thérapeutique ne permettent pas une médication optimale et/ou efficace.

WO 00/ 51596 et WO 02/ 062322 décrivent l'utilisation de la bétaine comme agent antithrombotique, seule ou en combinaison avec d'autres molécules. La thrombose étant une conséquence possible des artérites et se situe en aval de celles-ci. Ces documents n'enseignent donc pas que la bétaine permettrait de traiter les artérites, en particulier une ou des artérites dégénératives.

Aucun de ces documents ne décrit de façon suffisante l'utilisation de la bétaine afin de traiter la claudication intermittente. De manière surprenante l'inventeur a découvert les propriétés remarquables de la glycine bétaine pour traiter la claudication intermittente.

### L'invention

L'invention a pour objet l'utilisation de la glycine bétaine en tant que principe thérapeutiquement actif pour l'obtention d'un médicament pour le traitement de la claudication intermittente.

L'utilisation de la glycine bétaine en tant que principe thérapeutiquement actif pour le traitement des artérites ou pour l'obtention d'un médicament destiné au traitement des artérites dégénératives, en particulier pour l'obtention d'un médicament destiné à la prévention d'au moins une artérite dégénérative, voire pour l'obtention d'un médicament destiné à réduire ou limiter la vitesse de développement de dégénérescence d'artérite(s) dégénérative(s), ou encore mieux pour l'obtention d'un médicament destiné à arrêter ou bloquer le développement de dégénérescence d'artérite(s) dégénérative(s) et/ou pour l'obtention d'un médicament destiné à la rémission au moins partielle de dégénérescence d'artérite(s) dégénérative(s), est également divulguée.

Selon une forme de réalisation, l'invention a pour objet l'utilisation de la glycine bétaine pour la préparation d'une combinaison thérapeutique à usage oral pour le traitement de la claudication intermittente, ladite combinaison comprenant comme principe actif unique au moins 500 mg (avantageusement au moins 1000 mg, en particulier 2000 à 10000, de préférence de 3000 à 7000 mg de glycine bétaine sous une forme à libération immédiate) de glycine bétaine par dose unitaire et/ ou un mélange de glycine bétaine et d'aspirine comme principes actifs et/ou comme combinaison de principes actifs de glycine bétaine et d'aspirine.

Avantageusement, la composition ou combinaison unitaire à usage oral contient au moins 1000 mg de glycine bétaine sous une forme à libération immédiate, en particulier 2000 à 10000, de préférence de 3000 à 7000.

De préférence, la composition ou combinaison unitaire à usage oral contient au moins 250 mg de glycine bétaine sous une forme à libération contrôlée, plus spécifiquement au moins 250 mg de glycine bétaine sous une forme à libération contrôlée et au moins 250 mg de glycine bétaine sous une forme à libération immédiate.

Dans un aspect particulier de l'invention, une bétaine peut également être associée à un agent anti-cholestérol, telle une statine afin de traiter les pathologies décrites dans la présente demande. D'une manière optionnelle une composition pharmaceutique combinant aspirine/ bétaine/ agent anti-cholestérol est également décrite.

Les traitements suivants sont également divulgués:
- le traitement d'un patient souffrant d'une artérite, dans lequel on administre audit patient une quantité thérapeutiquement active de glycine bétaine pour le traitement de l'artérite.
- la prévention chez un patient l'apparition d'une artérite, dans laquelle on administre audit patient une quantité thérapeutiquement active de glycine bétaine pour prévenir ladite artérite.
- le traitement d'un patient souffrant d'une artérite, dans lequel on administre audit patient une quantité thérapeutiquement active de glycine bétaine pour le traitement d'artérite(s) dégénérative(s).
- le traitement d'un patient souffrant d'une artérite, dans lequel on administre audit patient une quantité thérapeutiquement active de glycine bétaine pour la prévention d'au moins une artérite dégénérative.
- le traitement d'un patient souffrant d'une artérite, dans lequel on administre audit patient une quantité thérapeutiquement active de glycine bétaine pour réduire ou limiter la vitesse de développement de dégénérescence d'artérite(s) dégénérative(s).
- le traitement d'un patient souffrant d'une artérite, dans lequel on administre audit patient une quantité thérapeutiquement active de glycine bétaine pour arrêter ou bloquer le développement de dégénérescence d'artérite(s) dégénérative(s).
- le traitement d'un patient souffrant d'une artérite, dans lequel on administre audit patient une quantité thérapeutiquement active de glycine bétaine pour la rémission au moins partielle de dégénérescence d'artérite(s) dégénérative(s).

Dans ces traitements, on administre avantageusement (en particulier par voie orale) au patient de manière journalière au moins 500 mg (avantageusement au moins 1000 mg, en particulier 2000 à 10000, de préférence de 3000 à 7000 mg de glycine bétaine sous une forme à libération immédiate) de glycine bétaine, avantageusement sous forme d'une dose unitaire et/ou d'une dose à prise unique journalière, et/ ou d'un mélange de glycine bétaine et d'aspirine comme principes actifs et/ou comme combinaison de principes actifs de glycine bétaine et d'aspirine. En particulier, on administre au patient de manière journalière au moins 1000 mg (avantageusement au moins 1500 mg, en particulier 2000 à 10000, de préférence de 3000 à 7000 mg de glycine bétaine sous une forme à libération immédiate) de glycine bétaine sous une forme à libération immédiate. Plus spécifiquement, on administre au patient de manière journalière au moins 250 mg de glycine bétaine sous une forme à libération contrôlée. Par exemple, on administre au patient de manière journalière au moins 250 mg, en particulier au moins 750 mg de glycine bétaine sous une forme à libération contrôlée et au moins 250 mg de glycine bétaine sous une forme à libération immédiate.

Des composés tels une bétaine de formule (CH₃)₃ N⁺ (CH₂) COO⁻ et ses sels pharmaceutiquement acceptables pourraient être utilisés afin de prévenir, de soulager et/ou traiter le syndrome de Raynaud ainsi que les atteintes physiopathologiques liées a cette affection,
ou
afin de prévenir, de soulager et/ou traiter la maladie de Burger ainsi que les atteintes physiopathologiques liées a cette affection,
ou
afin de prévenir, de soulager et/ou traiter les varices ainsi que les atteintes physiopathologiques liées a cette affection, ou
afin de prévenir, de soulager et/ou traiter les problèmes de circulation et de microcirculation liés aux jambes lourdes ainsi que les atteintes physiopathologiques liées a cette affection ou état,
ou
afin de prévenir, de soulager et/ou traiter les artérites temporales, les artérites giganto-cellulaire temporales, la maladie de Horton ainsi que les atteintes physiopathologiques liées a ces affections ou états telles que les céphalées, la claudication intermittente de la mâchoire lors des efforts de mastication, les douleurs linguales, la sensibilité du cuir chevelu, la cécité ainsi que l'induration des artères temporales, ou
afin de prévenir, de soulager et/ou traiter un oedème du pôle postérieur de l'oeil, la perte fonctionnelle de la vision, l'occlusion de l'artère centrale de la rétine ainsi que l'ischémie rétinienne,
afin de prévenir, de soulager et/ou traiter les troubles liés une vitesse de sédimentation accélérée,
ou
afin de prévenir, de soulager et/ou traiter les troubles liés aux algies d'origine inflammatoire comme dans la maladie de Horton,
ou afin de prévenir, de soulager et/ou traiter les troubles liés à l'impuissance masculine ainsi que les troubles génitaux d'origine artérielle pouvant se produire en cas d'obstruction vasculaire rendant l'érection, difficile, incomplète ou impossible;
ou
afin de prévenir, de soulager et/ou traiter les troubles liés à l'impuissance masculine ainsi que les troubles liés aux dysfonctions érectiles,
ou
afin de prévenir, de soulager et/ou traiter les troubles liés à la vasoconstriction du système circulatoire.

Bien que l'homocystéine n'a aucun effet causatif dans l'expression des artérites son rôle aggravant dans la maladie vasculaire est maintenant reconnu et dans un aspect particulier, l'utilisation d'au moins une bétaine de formule (CH₃)₃ N⁺ (CH₂) COO⁻ et ses sels pharmaceutiquement acceptables afin de traiter les artérites tout en normalisant et/ou abaissant les taux plasmatiques d'homocysteine chez un patient souffrant d'hyper homocysteinémie, est décrite.

Le terme artérite des membres inférieurs désigne en fait une artériopathie chronique obstructive. Ce terme regroupe les lésions de la paroi artérielle dont l'évolution est le rétrécissement ou sténose du calibre artériel. Les membres inférieurs sont souvent atteints par ce rétrécissement artériel qui provoque des douleurs lors de la marche et peut aller jusqu'à la gangrène et l'amputation.

### EXEMPLES

### Exemple 1

### Effet de la bétaine sur le sang non stimulé et perfusé sur des cellules endothéliales activées.

Nous avons étudié l'effet de la bétaine sur l'adhérence des plaquettes et la formation de thrombus sous un flux laminaire à hautes forces de cisaillement (shear stress 60 dynes /cm²) sur une lignée de cellules endothéliales micro vasculaires humaines (HMEC ⁻¹) dans des conditions de repos ou activées avec du PMA (200 µM, 45 minutes). Le sang humain héparinisé était pré labellisé avec de la mépacrine et exposé au véhicule seul ou à la bétaine (10, 20, 40, et 80 µg/ml) pendant 20 minutes avant la perfusion. A la fin des 3 minutes de perfusion, les cellules ont été fixées et la surface couverte par les thrombi a été calculée par analyse d'images de la fluorescente des thrombi acquises par microscopie confocale.

### Méthodes expérimentales

### Adhérence des plaquettes et formation du thrombus.

L'essai d'adhérence des plaquettes a été réalisé selon les méthodes décrites par Alevriadou et al. 1993 et Boccardo et al. 1997, avec quelques modifications mineures. Le sang humain prélevé sur héparine (concentration finale 10 IU/ml) est perfusé dans la chambre de flux laminaire en utilisant une pompe-seringue. Cette chambre de flux est thermostatisée à 37° C et une de ses parois en verre est revêtue avec un monolayer de cellules endothéliales sur lesquelles le sang traité à la bétaine est perfusé.

### Chambre de perfusion.

Les dimensions de la chambre de perfusion (30 mm de long, 1 mm de large, et 150 µm d'épaisseur) permettent d'obtenir une large gamme de taux de cisaillements à de bas débits de sang. Les conditions de flux sont bien caractérisées pour cette configuration et permettent d'être laminaires avec un nombre de Reynolds très bas (<10). Une évaluation précise des forces de cisaillement sur la surface d'adhésion est également réalisée en utilisant un programme d'analyse de la dynamique des fluides (CFD package FIDP, fluid dynamic international, Evanston, II) qui permet de vérifier l'effet de l'entrée et de la sortie sur les profils de vélocité du flux.

### Essai d'adhérence sur endothélium.

Une lignée humaine de cellules endothéliales micro vasculaires d'origine dermale (HMEC-1) a été cultivée dans MCDB 131 supplémenté avec 10% de FCS, 1µg/ml d'hydrocortisone, 100 U/ml de pénicilline, 100 µg/ml de streptomycine, 2 mM de glutamine et 50 µg ml de facteur de croissance de cellules endothéliales. Pour les expériences, les cellules ont été étalées sur un coverslip de verre et à la confluence ont été stimulées avec du PMA (phorbol 12-myristate 13-acetate) dans des conditions statiques. A la fin de l'incubation les cellules ont été perfusées avec le sang humain. Le sang héparinisé a été incubé avec de la mépacrine 10 µM qui donne une teinte fluorescente (quinacrine dihydrochloride BP; Sigma Chimique, Louis de St. MO). La mépacrine se concentre dans les granules denses des plaquettes et dans les granules des leucocytes et à cette concentration n'a pas d'effet sur la fonction normale de la plaquette ; toute fluorescence dans les érythrocytes étant capturée par l'hémoglobine.

Le sang est alors pré incubé 5 minutes à 37° C avant la perfusion. Le système ayant été rempli avec du PBS pH 7,3, la perfusion avec le sang est commencée et maintenue au débit constant de 1500 sec-1 (correspondant à un shear stress de 60 dynes/cm² c'est-à-dire aux conditions artérielles ou que l'on pourrait retrouver dans des conditions pathologiques de sténose ou d'artérites). Après 3 minutes, la perfusion est arrêtée et la paroi avec le monolayer de cellules endothéliales est déshydratée et fixée dans de l'acétone pendant 20 minutes. Les images des plaquettes formant le thrombus sur la surface de cellules endothéliales sont alors acquises par un microscope confocal à laser inversé (Insight plus; Meridian Instruments Inc, Okemos, MI). La surface occupée par chaque thrombus a été évaluée en utilisant un logiciel de détection Image 1.61 (NIH Bethesda, MD). 15 champs, systématiquement digitalisés le long de la surface d'adhérence ont été saisis.

### Résultats

Effet de la bétaine sur la formation de thrombus sur HMEC-1 stimulées par le PMA sous des conditions flux (60 dynes/cm²)

| Exp. n° | contrôle | PMA 200 nM 45' | PMA+ bétaine 20 µg/ml | PMA+ bétaine 40 µg/ml | PUMA+ bétaine 80 µg/ml |
|---|---|---|---|---|---|
| #4 | 1649 | 4822 | 3059 | 3947 | 2451 |
| #6 | 1139 | 6243 | 1290 | 5146 | 1852 |
| #9 | 680 | 11843 | 1436 | 2470 | 4150 |
| #10 | 465 | 6538 | - | 1972 | 1077 |
| #12 | 845 | 5448 | - | 2795 | - |
| **Moyenne** | **955** | **6979** | **1928** | **3266** | **2388** |
| ±Dev. Std | ± 459 | ± 2880° | ± 982** | ± 1277* | ± 1305** |

Data are expressed as area covered by thrombi (µm²/field)
HMEC-1: human microvascular endothelial cell line
PMA: phorbol 12-myristate 13-acetate
Blood was incubated with bétaine for 20 minutes
° p < 0.01 Vs control * p < 0.05, ** p< 0.01 Vs PMA

### Commentaires

Les cellules endothéliales stimulées par le PMA expriment le TNF α (Tumor Necrosis Factor Alpha) qui lui-même induit l'expression de diverses molécules telles que les prostaglandines, les I-CAM, les interleukines et diverses cytokines qui sont toutes des marqueurs de l'inflammation. L'intérêt de cette expérimentation réside dans le fait que les conditions d'une artérite, notamment l'inflammation des cellules endothéliales et de là des parois endothéliales, sont réunies.

En effet les différents acteurs d'une artérite sont présents soit : le sang, la paroi endothéliale enflammée et les forces de cisaillements. Les cellules témoins, c'est-à-dire non enflammées puisque non stimulées par le PMA montrent une propension limitée à adhérer les plaquettes malgré des forces de cisaillement élevées et il n'y a pas de formation de thrombus donc pas de conditions oblitérantes. Par contre dès que ces cellules sont enflammées leur propension à former des thrombi est remarquable et mime parfaitement les conditions pathologiques d'une artérite. Dans ce modèle, la bétaine en ramenant à la normale l'adhésivité des cellules endothéliales agit sur l'expression de diverses molécules telles que les prostaglandines, les I-CAM, les interleukines ainsi que diverses cytokines et montre son activité sur ces marqueurs de l'inflammation et de là sur l'inflammation. Cette activité anti-inflammatoire de la bétaine permet d'envisager son usage dans le traitement des artérites, qu'elles soient oblitérantes ou non.

### Exemple 2

### Effet de la betaine sur la thrombine et la viscosité du sang

A mesure que la viscosité du sang augmente, l'écoulement du sang diminue dans tout le corps, créant l'anoxie (manque de l'oxygène) et la privation nutritive dans divers secteurs du corps. Ceci explique également pourquoi la thérapie de bétaine est efficace en augmentant l'écoulement du sang car la viscosité du sang diminue. Ainsi, les patients obtiennent plus de soulagement de leurs symptômes avec cette thérapie.

Si le mécanisme de coagulation ne s'arrête pas correctement, alors il y a génération continue de thrombine et formation soluble de fibrine, ayant pour résultat la viscosité accrue du sang et l'écoulement diminué du sang dans l'organisme.

En outre, in vitro, la bétaine s'oppose aux effets induits par l'interleukine 1 et le Tumor Necrosis Factor sur le polynucléaire neutrophile, à savoir le ralentissement du déplacement, l'augmentation de l'adhésivité et la libération excessive d'ions superoxydes et peroxydes d'hydrogène que ces médiateurs entraînent.

De même, la bétaine s'oppose à la baisse de la déformabilité érythrocytaire, perturbation physiologique dont le rôle a été mis en évidence dans de nombreux processus pathologiques, en particulier au cours des artériopathies des membres inférieurs. Il résulte alors de l'administration de la glycine bétaine une facilitation de la circulation des globules rouges, la microcirculation étant améliorée, sans augmenter le débit au niveau des zones saines aux dépens des zones ischémiées.

### a) Agrégation in vitro sur sang total animal induite par la thrombine

Pour démontrer l'effet de la bétaine sur la thrombine nous avons réalisé des tests d'agrégation sur sang total. Cette technique sur sang total présente l'avantage en ce que tous les éléments du sang sont présent notamment les globules rouges et le globules blancs, ces derniers étant des acteurs importants dans les phénomènes d'inflammation. Par les phénomènes de déformabilité les érythrocytes sont également des acteurs puissants dans les pathologies liées aux artérites.

### Protocole.

Le sang de rats Wistar prélevé sur citrate et aliquoté en 2 échantillons pour chaque animal, un échantillon est mixé avec du liquide physiologique (témoin) l'autre avec une solution de bétaine à la concentration de 5 mg/ml afin d'obtenir une concentration finale de 50 µg/ml de bétaine dans le sang (traité). (10 µL de solution pour 990 µL de sang). Les échantillons sont alors incubés pendant 20 min. à 37° C sous légère agitation puis la thrombine est ajoutée (1 IU. /ml) et les tests réalisés avec un Agrégomètre Chronolog 500.

### Résultats

Les amplitudes sont exprimées en ohm et les vélocités en ohm/min.

| | **NaCl 0, 9 %** | | **Bétaine 50 µg/ ml** | |
|---|---|---|---|---|
| | Amplitude Ω | Vélocité Ω / min. | Amplitude Ω | Vélocité Ω / min. |
| Rat 1 | 22 | 14 | 5 | 7 |
| Rat 2 | 17 | 18 | 3 | 5 |
| Rat 3 | 25 | 16 | 12 | 7 |
| Rat 4 | 18 | 16 | 8 | 5 |
| Rat 5 | 24 | 18 | 4 | 3 |
| **Moyenne** | **21,2 ± 3,56** | **16,4 ± 1,67** | **6,4 ± 3,65** | **5,4 ± 1,67** |

Les résultats montrent la puissante activité de la bétaine vis-à-vis de la thrombine. Cette dernière intervenant également dans les pathologies décrites plus haut notamment la viscosité du sang et les phénomènes inflammatoires, il apparaît que l'utilisation thérapeutique de la bétaine peut être envisagée pour ces pathologies ainsi que toutes les autres pathologies impliquant la thrombine.

### b) Effet de la bétaine chez des volontaires sains après administration orale

Le but de cette étude est d'évaluer l'évolution de divers paramètres hématologiques après aministration orale de 2 x 3 gr. de bétaine monohydrate (Betafin AP) pendant 4 jours à des volontaires sains.

### Protocole

Les tests on été conduits à l'Hôpital de la Citadelle à Liège, Service d'Hématologie Clinique. Les volontaires ont donné leur consentement écrit avant les expériences.

Les prélèvements sanguins ont été effectués par ponction veineuse au bras chez les donneurs à jeun sur Na-citrate le premier jour à 9 heures (j1) afin de déterminer les valeurs basales. Juste après les prélèvements, les volontaires ont pris une première dose de 3 gr. de bétaine avec leur petit déjeuner. La dose suivante le même jour à 12 heures d'intervalle. Les jours 3 suivants les prises se sont poursuivies matin et soir ainsi qu'une dernière prise de 3 gr. à jeun, 2 heures avant les prélèvements sanguins le dernier jour (j5).

### Résultats.

| | Fibrinogène | | Thrombine/ antithrombine | |
|---|---|---|---|---|
| | Jour 1 | Jour 5 | Jour 1 | Jour 5 |
| Donneur 1 | 3,8 | 3,3 | 2,5 | 2,1 |
| Donneur 2 | 3,3 | 3,1 | 2,9 | 2,2 |
| Donneur 3 | 3,4 | 3 | 2,9 | 2,2 |
| Donneur 4 | 2,9 | 2,2 | 3,2 | 2,6 |
| Donneur 5 | 3,1 | 2,9 | 3,1 | 2,2 |
| **Moyenne** | **3,3** | **2,9** | **2,92** | **2,26** |
| ***Variation à J 5*** | ***-12 %*** | | ***-23 %*** | |

### Commentaires.

Une baisse significative intervient chez tous les volontaires au niveau des marqueurs de la coagulation et de la viscosité du sang. Il apparaît que l'administration de la bétaine réduit le fibrinogène et en fluidifiant le sang permet une meilleure circulation sanguine. Cette réduction de la viscosité sanguine permet de pouvoir mieux irriguer les zones ischémiées dans les pathologies liées aux artérites. Au cours de cette même étude chez 3 sujets sur 5 la liaison du facteur von Willebrand au collagène a évolué vers le bas indiquant l'activité thérapeutique de la bétaine sur l'activité fonctionnelle du facteur von Willebrand et ainsi des pathologies liées à ce facteur.

### c) Génération de thrombine in vitro sur sang humain

### Protocole

Ces tests ont été réalisés à l'Institut Cardiovasculaire de Maastricht. Le sang de 5 volontaires sains a été prélevé sur Na-citrate (3,8 %, 1 :10) et centrifugé afin d'obtenir du PRP. Ce PRP est ensuite incubé pendant 20 minutes à une solution isotonique de bétaine (Ph 7) ou à du liquide physiologique, chaque donneur étant son propre témoin. La concentration finale de bétaine dans les PRP testés étant de 100 µg/ml.

On a déterminé alors la génération de thrombine grâce au Thrombogram ®.

Les résultats obtenus montrent qu'en présence de bétaine la génération de thrombine est abaissée de 15 à 25 % en fonction du donneur. Cette activité inhibitrice de la bétaine sur la génération de thrombine étant comparable à celles obtenues, dans ce modèle, à celles obtenues après ingestion d'aspirine ou après administration expérimentale d'anti-glycoprotéines Ib. Le fait de réduire la génération de thrombine permet de réduire la réactivité du sang ainsi que sa coagulabilité et sa viscosité. Cette activité inhibitrice de la bétaine en permettant une meilleure circulation sanguine, le sang étant moins visqueux, réduit les artérites et les atteintes liées aux artérites. L'usage thérapeutique de la bétaine pouvant également être envisagée dans toutes les pathologies impliquant une génération accrue de la thrombine.

### Exemple 3

### Challenge inflammatoire provoqué par un stress oxydatif sur la paroi endothéliale, effet de la bétaine.

Les artérites étant également des conditions pathologiques où le stress oxydatif s'exprime sur les parois vasculaires, il était intéressant d'évaluer l'effet de la bétaine sur un modèle de radicaux libres. Dans ce modèle la paroi endothéliale suite à l'agression des radicaux libres présente le même type de réactions que dans les pathologies de l'invention.

### Protocole expérimental

Des hamsters pesant entre 120 et 130 grammes ont été utilisés dans ces tests. On injecte à ces animaux une solution de rose Bengale avant d'exposer leurs veines fémorales à une source de lumière polarisée verte afin de produire in situ des radicaux libres. La lumière verte polarisée dégrade le rose Bengale ce qui produit des radicaux libres connus pour agresser la paroi endothéliale et produire des conditions identiques à celles rencontrées dans l'inflammation, les artérites et de là la thrombose. Ce modèle expérimental dépeint parfaitement l'interaction entre l'endothélium qui suite à ce stress oxydatif devient une surface procoagulante et les éléments du sang. On quantifie la formation du thrombus par la saisie d'images toutes les 10 secondes de la veine fémorale pendant 40 minutes (240 images par expérience). Le calcul de la quantité de lumière blanche de chacune des 240 images permet de quantifier la taille et la progression des thrombus. L'intensité de lumière blanche correspond au nombre de plaquettes dans le thrombus qui comprend aussi des leucocytes et des érythrocytes.

Les substances étudiées sont injectées en bolus (22 ou 66 mg/kg) par voie intraveineuse à T0, suivie d'une infusion continue (22 ou 66 mg/kg) pendant 40 minutes.

### Résultats

| | **Contrôle** | **22 mg/kg** | **66 mg/kg** |
|---|---|---|---|
| Hamster 1 | 413641 | 123074 | 70729 |
| # 2 | 422820 | 47271 | 43315 |
| # 3 | 449691 | 188041 | 56714 |
| # 4 | 379908 | 121924 | 140621 |
| # 5 | 452849 | 135688 | 162353 |
| # 6 | 220109 | 477299 | 162477 |
| # 7 | 359792 | 220730 | - |
| # 8 | 268123 | 88407 | - |
| **Moyenne** | **370 867** | **175304** | **106035** |

### Commentaires

Une baisse de - 53 % et - 71 % à 22 mg/kg et 66 mg/kg respectivement dans la luminescence enregistrée. Il apparaît que la paroi endothéliale en présence de bétaine développe moins de propriétés procoagulantes vu que les thrombus formés sont beaucoup moins importants. Dans ce modèle il a été démontré que les radicaux libres générés par la polarisation du rose Bengale n'avaient pas ou peu d'influence sur l'activité plaquettaire et/ou la rhéologie du sang, ce qui implique que la bétaine exerce une influence directe sur l'adhésivité de la paroi endothéliale. En effet, les radicaux libérés en agressant la paroi endothéliale créent les conditions de diverses pathologies telles que l'inflammation dégénérative, l'inflammation, le cancer, l'inflammation oblitérante ainsi que les conditions procoagulantes liées aux infections d'ordre viral et/ou bactérien. L'activité de la bétaine dans ce modèle animal in vivo démontre son efficacité sur les pathologies impliquant l'agression de la paroi endothéliale par les radicaux libres. Le stress oxydatif sur la paroi des vaisseaux de ces expérimentations est similaire à celui rencontré dans artérites.

### Exemple 4

Mesure in vitro de la viscosité et de la coagulabilité du sang dans le système TEG® après ajout de bétaine.

### Protocole

Le sang de 6 volontaires sains a été prélevé, supplémenté de diverses concentrations de bétaine et directement (moins de quatre minutes après le prélèvement) évalué dans le Thromboélastogramme TEG® (Haemoscope Corporation USA). Les concentrations finales de bétaine étudiées étaient les suivantes 0, 100, 250 et 500 µg/ml.

### Résultats

| Concentration Betaine | R (mm) | RK (mm) | K (mm) | MA (mm) | Angle α (°) |
|---|---|---|---|---|---|
| 0 µg/ml (NaCl 0.9%) | 30 | 34 | 4 | 68 | 63° |
| 100 µg/ml | 40 | 50 | 10 | 64 | 39° |
| 250 µg/ml | 46 | 55 | 9 | 61 | 45° |
| 500 µg/ml | 95 | 111 | 16 | 68 | 34° |

### Commentaires

L'effet de la bétaine est concentration dépendant et affecte pratiquement tous les paramètres du TEG. Une activité anticoagulante est démontrée dans ces expérimentations, ainsi qu'un effet très significatif sur le temps de formation initial de fibrine (r) et de là de la viscosité sanguine. Une activité notable est également démontrée sur l'Angle α qui traduit la rapidité de formation du réseau de fibrine dans le sang et de là sa viscosité et la lenteur avec laquelle il pourrait s'écouler dans les vaisseaux. La bétaine montre son effet sur ces paramètres et en rendant le sang moins visqueux tend à faciliter son écoulement et réduire son adhésion dans le système vasculaire.

Dans ce système en réduisant l'Angle α la bétaine démontre également son activité fibrinolytique en fragilisant le caillot formé et de là en permettant sa destruction (lyse) plus rapide.

L'intérêt de cette expérimentation est qu'elle utilise le sang natif, c'est-à-dire sans ajout d'autre anticoagulant que la bétaine et permet ainsi d'être le plus proche possible des conditions physiologiques que l'on pourrait rencontrer in vivo après administration de la bétaine.

### Exemple 5

### Test clinique sur tapis roulant

Chez 7 malades souffrant d'artérite et de claudication intermittente, ne prenant pas d'autre médication que l'aspirine (75 à 325 mg/jour) il a été prescrit une dose de 4 grammes/jour par voie orale de bétaine monohydrate (Betafin ® Finnsugar) divisés en deux prises par jour. Le traitement a duré 4 semaines.

Les malades ont subi un test de marche sur tapis roulant le premier et le dernier jour du traitement afin d'évaluer l'effet de la bétaine.

Le test de marche sur tapis roulant est standardisé, la vitesse du tapis roulant est de 3,2 Kmm et la pente de 12 %. Il permet d'évaluer le périmètre de début de la claudication puis le périmètre maximal d'arrêt de la marche, c'est-à-dire le moment où la douleur empêche le patient de continuer à marcher.

### Résultats

Chez 5 patients on a noté une augmentation du périmètre début de la claudication de 25% et une augmentation du périmètre total de marche de plus de 30% grâce à un recul de l'apparition de la douleur et/ou à une marche plus efficiente. On a noté également un recul du temps de l'apparition des premières gênes de 20%.

Chez les 2 autres l'effet étant moins significatif l'augmentation du périmètre total de marche se situant à près de 15%.

Le questionnaire appliqué à chacun de ces volontaires a permis de dégager une tendance générale de confort accru ainsi qu'une meilleure perception physique de l'effort.

## Revendications

1. Utilisation de la glycine betaine ou d'un sel thérapeutiquement actif de cellc-ci en tant que principe thérapeutiquement actif pour la préparation d'une composition pharmaceutique pour le traitement de la claudication intermittente.

2. Utilisation de la glycine betaine ou d'un sel thérapeutiquement actif de celle-ci en tant que principe thérapeutiquement actif pour la préparation d'une composition pharmaceutique à usage oral pour le traitement de la claudication intermittente, ladite composition comprenant comme principe actif unique une quantité thérapeutiquement active de glycine betaine par dose unitaire.

3. Utilisation de la glycine betaine ou d'un sel thérapeutiquement actif de celle-ci en tant que principe thérapeutiquement actif pour la préparation d'une composition ou combinaison pharmaceutique pour accroitre le périmètre total de marche chez un patient souffrant de claudication intermittente.

4. Utilisation suivant la revendication 3 dans laquelle la glycine betaine ou un sel pharmaceutique de celle-ci est utilisée en tant que principe thérapeutiquement actif pour la préparation d'une composition ou combinaison pharmaceutique pour accroitre le périmètre total de marche, ledit accroissement étant mesuré par un test standardisé de marche sur tapis roulant, dont la vitesse est de 3,2 Km/h et la pente de 12 %.

5. Utilisation suivant la revendication 3 dans laquelle la glycine bétaine ou un sel pharmaceutique de celle-ci est utilisée en tant que principe thérapeutiquement actif pour la préparation d'une composition ou combinaison pharmaceutique pour accroitre le périmètre total de marche de plus de 30%.

6. Utilisation de la glycine betaine ou d'un sel thérapeutiquement actif de celle-ci en tant que principe thérapeutiquement actif pour la préparation d'une composition ou combinaison pharmaceutique pour accroitre le périmètre de début de claudication chez un patient souffrant de claudication intermittente.

7. Utilisation suivant la revendication 6 dans laquelle la glycine betaine ou un sel pharmaceutique de celle-ci est utilisée en tant que principe thérapeutiquement actif pour la préparation d'une composition ou combinaison pharmaceutique pour accroitre le périmètre de début de claudication, ledit accroissement étant mesuré par un test standardisé de marche sur tapis roulant, dont la vitesse est de 3,2 Km/h et la pente de 12%.

8. Utilisation suivant la revendication 6 dans laquelle la glycine betaine ou un sel pharmaceutique de ccllc-ci est utilisée en tant que principe thérapeutiquement actif pour la préparation d'une composition ou combinaison pharmaceutique pour accroître le périmètre de début de claudication d'au moins 25%.

9. Utilisation suivant la revendication 3 ou 6 dans laquelle la glycine betaine ou un sel pharmaceutique de celle-ci est utilisée en tant que principe thérapeutiquement actif pour la préparation d'une composition ou combinaison pharmaceutique pour accroitre le périmètre total de marche et le périmètre du début de la claudication, tout en assurant un recul de l'apparition de la douleur et/ou une marche plus efficiente.

10. Utilisation suivant la revendication 3 ou 6 dans laquelle la glycine betaine ou un sel pharmaceutique de celle-ci est utilisée en tant que principe thérapeutiquement actif pour la préparation d'une composition ou combinaison pharmaceutique pour accroitre le périmètre total de marche et le périmètre du début de la claudication, tout en assurant un recul du temps de l'apparition des premières gênes de 20%.

11. Utilisation suivant la revendication 3 ou 6 dans laquelle la glycine betaine ou un sel pharmaceutique de celle-ci est utilisée en tant que principe thérapeutiquement actif pour la préparation d'une composition ou combinaison pharmaceutique pour accroître le périmètre total de marche et le périmètre du début de la claudication, tout en assurant une tendance générale de confort accru.

12. Utilisation suivant la revendication 3 ou 6 dans laquelle la glycine betaine ou un sel pharmaceutique de celle-ci est utilisée en tant que principe thérapeutiquement actif pour la préparation d'une composition ou combinaison pharmaceutique pour accroitre le périmètre total de marche et le périmètre du début de la claudication, tout en assurant une meilleure perception physique de l'effort.

13. Utilisation suivant l'une quelconque des revendications précédentes, de la glycine betaine pour la préparation d'une composition ou combinaison thérapeutique à usage oral pour le traitement de la claudication intermittent, ladite composition ou combinaison comprenant comme principe actif unique au moins 500 mg de glycine betaine par dose .unitaire et/ ou un mélange de glycine betaine et d'aspirine comme principes actifs et/ou comme combinaison de principes actifs de glycine betaine et d'aspirine.

14. Utilisation selon la revendication 13 pour la préparation d'une composition ou combinaison unitaire à usage oral, **caractérisée en ce qu'**elle contient au moins 1000 mg de glycine betaine sous une forme à libération immédiate.

15. Utilisation selon la revendication 13 pour la préparation d'une composition ou combinaison unitaire à usage oral, **caractérisée en ce qu'**elle contient au moins 250 mg de glycine bétaine sous une forme à libération contrôlée.

16. Utilisation scion la revendication 13 pour la préparation d'une composition ou combinaison unitaire à usage oral, **caractérisée en ce qu'**elle contient au moins 250 mg de glycine betaine sous une forme à libération contrôlée et au moins 250 mg de glycine betaine sous une forme à libération immédiate.

17. Utilisation de la glycine bétaine pour la préparation d'une composition thérapeutique à usage oral pour le traitement de la claudication intermittente, ladite composition comprenant comme principe actif unique au moins 500 mg de glycine bétaine par dose unitaire.

## Claims

1. Use of glycine betaine or a therapeutically active salt thereof as therapeutically active principle for the preparation of a pharmaceutical composition for the treatment of intermittent claudication.

2. Use of glycine betaine or a therapeutically active salt thereof as therapeutically active principle for the preparation of a pharmaceutical composition for oral use for the treatment of intermittent claudication, said composition comprising as unique active principle a therapeutically effective amount of glycine betaine by unitary dose.

3. Use of glycine betaine or a therapeutically active salt thereof as therapeutically active principle for the preparation of a pharmaceutical composition or combination for increasing the total walking perimeter in a patient suffering of intermittent claudication.

4. Use according to claim 3 wherein glycine betaine or a therapeutically active salt thereof is used as therapeutically active principle for the preparation of a pharmaceutical composition or combination for increasing the total walking perimeter, said increase being measured by a standardised walking test on a treadmill set at a speed of 3.2 km/h and at 12% grade inclination.

5. Use according to claim 3 wherein glycine betaine or a therapeutically active salt thereof is used as therapeutically active principle for the preparation of a pharmaceutical composition or combination for increasing the total walking perimeter by more than 30%.

6. Use of glycine betaine or a therapeutically active salt thereof as therapeutically active principle for the preparation of a pharmaceutical composition or combination for increasing the perimeter of initial claudication in a patient suffering of intermittent claudication.

7. Use according to claim 6 wherein glycine betaine or a therapeutically active salt thereof is used as therapeutically active principle for the preparation of a pharmaceutical composition or combination for increasing the perimeter of initial claudication, said increase being measured by a standardised walking test on a treadmill set at a speed of 3.2 km/h and at 12% grade inclination.

8. Use according to claim 6 wherein glycine betaine or a therapeutically active salt thereof is used as therapeutically active principle for the preparation of a pharmaceutical composition or combination for increasing the perimeter of initial claudication at least by 25%.

9. Use according to claim 3 or 6 wherein glycine betaine or a therapeutically active salt thereof is used as therapeutically active principle for the preparation of a pharmaceutical composition or combination for increasing the total walking perimeter and the perimeter of initial claudication, whilst assuring a decline of the onset of pain and/or a more efficient walking.

10. Use according to claim 3 or 6 wherein glycine betaine or a therapeutically active salt thereof is used as therapeutically active principle for the preparation of a pharmaceutical composition or combination for increasing the total walking perimeter and the perimeter of initial claudication, whilst assuring a decline of the time of onset of the first discomforts by 20%.

11. Use according to claim 3 or 6 wherein glycine betaine or a therapeutically active salt thereof is used as therapeutically active principle for the preparation of a pharmaceutical composition or combination for increasing the total walking perimeter and the perimeter of initial claudication, whilst assuring a general feeling of improved comfort.

12. Use according to claim 3 or 6 wherein glycine betaine or a therapeutically active salt thereof is used as therapeutically active principle for the preparation of a pharmaceutical composition or combination for increasing the total walking perimeter and the perimeter of initial claudication, whilst assuring a better perception of effort.

13. Use according to anyone of the preceding claims, of glycine betaine for the preparation of a pharmaceutical composition or combination for oral use for the treatment of intermittent claudication, said composition or combination comprising as unique active principle at least 500 mg of glycine betaine by unitary dose and/or a mix of glycine betaine and aspirin as active principles and/or a combination of active principles of glycine betaine and aspirin.

14. Use according to claim 13 for the preparation of an unitary composition or combination for oral use, **characterised in that** it contains at least 1000 mg of glycine betaine in immediate release form.

15. Use according to claim 13 for the preparation of an unitary composition or combination for oral use, **characterised in that** it contains at least 250 mg of glycine betaine in controlled release form.

16. Use according to claim 13 for the preparation of an unitary composition or combination for oral use, **characterised in that** it contains at least 250 mg of glycine betaine in controlled release form and at least 250 mg of glycine betaine in immediate release form.

17. Use of glycine betaine for the preparation of a therapeutical composition for oral use for the treatment of intermittent claudication, said composition comprising as unique active principle at least 500 mg of glycine betaine by unitary dose.

## Patentansprüche

1. Verwendung von Glycin-Betain oder eines therapeutisch wirksamen Salzes desselben als therapeutischer Wirkstoff für die Herstellung einer pharmazeutischen Zusammensetzung zur Behandlung von Claudicatio intermittens.

2. Verwendung von Glycin-Betain oder eines therapeutisch wirksamen Salzes desselben als therapeutischer Wirkstoff für die Herstellung einer pharmazeutischen Zusammensetzung zum oralen Gebrauch zur Behandlung von Claudicatio intermittens, wobei die Zusammensetzung als einzigen Wirkstoff eine therapeutisch wirksame Menge von Glycin-Betain pro Einheitsdosis umfasst.

3. Verwendung von Glycin-Betain oder eines therapeutisch wirksamen Salzes desselben als therapeutischer Wirkstoff für die Herstellung einer pharmazeutischen Zusammensetzung oder Kombination, um die Gesamtgehstrecke bei einem Patienten zu erhöhen, der an Claudicatio intermittens leidet.

4. Verwendung nach Anspruch 3, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke zu erhöhen, wobei die Erhöhung durch einen standardisierten Gehtest auf einem Laufband gemessen wird, dessen Geschwindigkeit 3,2 km/h und dessen Anstieg 12 % beträgt.

5. Verwendung nach Anspruch 3, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke um mehr als 30 % zu erhöhen.

6. Verwendung von Glycin-Betain oder eines therapeutisch wirksamen Salzes desselben als therapeutischer Wirkstoff für die Herstellung einer pharmazeutischen Zusammensetzung oder Kombination, um die Gehstrecke bis zum Auftreten der Claudicatio bei einem Patienten zu erhöhen, der an Claudicatio intermittens leidet.

7. Verwendung nach Anspruch 6, wobei das Glycin-Betain oder ein pharmazeutisches Salz desselben als therapeutischer Wirkstoff für die Herstellung einer pharmazeutischen Zusammensetzung oder Kombination verwendet wird, um die Gehstrecke bis zum Auftreten der Claudicatio zu erhöhen, wobei die Erhöhung durch einen standardisierten Gehtest auf einem Laufband gemessen wird, dessen Geschwindigkeit 3,2 km/h und dessen Anstieg 12 % beträgt.

8. Verwendung nach Anspruch 6, wobei das Glycin-Betain oder ein pharmazeutisches Salz desselben als therapeutischer Wirkstoff für die Herstellung einer pharmazeutischen Zusammensetzung oder Kombination verwendet wird, um die Gehstrecke bis zum Auftreten der Claudicatio um mindestens 25 % zu erhöhen.

9. Verwendung nach Anspruch 3 oder 6, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke und die Gehstrecke bis zum Auftreten der Claudicatio zu erhöhen und gleichzeitig einen Rückgang des Auftretens der Schmerzen und/oder ein effizienteres Gehen sicherzustellen.

10. Verwendung nach Anspruch 3 oder 6, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke und die Gehstrecke bis zum Auftreten der Claudicatio zu erhöhen und gleichzeitig eine Verlängerung der Zeit bis zum Auftreten der ersten Beschwerden um 20 % sicherzustellen.

11. Verwendung nach Anspruch 3 oder 6, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke und die Gehstrecke bis zum Auftreten der Claudicatio zu erhöhen und gleichzeitig eine allgemeine Tendenz des erhöhten Komforts sicherzustellen.

12. Verwendung nach Anspruch 3 oder 6, wobei das Glycin-Betain oder ein pharmazeutisches Salz desselben als therapeutischer Wirkstoff für die Herstellung einer pharmazeutischen Zusammensetzung oder Kombination verwendet wird, um die Gesamtgehstrecke und die Gehstrecke bis zum Auftreten der Claudicatio zu erhöhen und gleichzeitig eine bessere physische Wahrnehmung der Anstrengung sicherzustellen.

13. Verwendung nach einem der vorhergehenden Ansprüche von Glycin-Betain für die Herstellung einer therapeutischen Zusammensetzung oder Kombination zum oralen Gebrauch zur Behandlung von Claudicatio intermittens, wobei die Zusammensetzung oder Kombination als einzigen Wirkstoff mindestens 500 mg Glycin-Betain pro Einheitsdosis und/oder ein Gemisch von Glycin-Betain und Aspirin als Wirkstoffe und/oder als Wirkstoffkombination von Glycin-Betain und Aspirin umfasst.

14. Verwendung nach Anspruch 13 für die Herstellung einer einheitlichen Zusammensetzung oder Kombination zum oralen Gebrauch, **dadurch gekennzeichnet, dass** sie mindestens 1000 mg Glycin-Betain in einer Form mit sofortiger Freisetzung enthält.

15. Verwendung nach Anspruch 13 für die Herstellung einer einheitlichen Zusammensetzung oder Kombination zum oralen Gebrauch, **dadurch gekennzeichnet, dass** sie mindestens 250 mg Glycin-Betain in einer Form mit kontrollierter Freisetzung enthält.

16. Verwendung nach Anspruch 13 für die Herstellung einer einheitlichen Zusammensetzung oder Kombination zum oralen Gebrauch, **dadurch gekennzeichnet, dass** sie mindestens 250 mg Glycin-Betain in einer Form mit kontrollierter Freisetzung und mindestens 250 mg Glycin-Betain in einer Form mit sofortiger Freisetzung enthält.

17. Verwendung von Glycin-Betain für die Herstellung einer therapeutischen Zusammensetzung zum oralen Gebrauch zur Behandlung von Claudicatio intermittens, wobei die Zusammensetzung als einzigen Wirkstoff mindestens 500 mg Glycin-Betain pro Einheitsdosis umfasst.
